# EUROPEAN PATENT SPECIFICATION

(11) **EP 2 793 626 B1**
(45) Date of publication and mention of the grant of the patent: **30.05.2018**
(21) Application number: 12809321.8
(22) Date of filing: 20.12.2012
(51) Int. Cl.: A24D 3/04

(54) **SMOKING ARTICLES AND OTHER FLOW DELIVERY ARTICLES**
RAUCHARTIKEL UND ANDERE FLUSSFREISETZUNGSARTIKEL
OBJETS POUR LE TABAGISME ET AUTRES OBJETS DE DISTRIBUTION DE FLUX

(30) Priority: 20.12.2011 GB 201121922; 20.12.2011 GB 201121920
(43) Date of publication of application: 29.10.2014
(73) Proprietor: British American Tobacco (Investments) Limited, London WC2R 3LA (GB)
(72) Inventor: FROBISHER, Paul, Clevedon BS21 6UW (GB); NEWNHAM, Michael, Clevedon BS21 6UW (GB); AWTY, Edward, London WC2R 3LA (GB); NICHOLLS, Jane, London WC2R 3LA (GB); NANDRA, Charanjit, London WC2R 3LA (GB); BOAST, David, Chippenham Wiltshire SN14 8DL (GB); SMITH, Simon, Cambridge CB4 0DW (GB); ABERCROMBIE, Stuart, Cambridge CB4 0DW (GB)
(74) Representative: EIP
(86) International application number: PCT/GB2012/053202
(87) International publication number: WO 2013/093470

(56) References cited:
- EP-A1- 2 380 618
- WO-A1-2005/053444
- WO-A1-2010/010391
- WO-A1-2010/038233
- WO-A2-2006/129304
- US-A- 4 638 820
- US-A- 4 677 995
- US-A1- 2004 030 508

## Description

### Field

This disclosure relates to a flow delivery article such as a smoking article. In particular, but not exclusively, the disclosure relates to a flow delivery article with pulsed delivery.

### Background

Known filter cigarettes include a tobacco rod component and one or more filter components which are joined to the tobacco rod by a wrapper known as a tipping paper. Such cigarettes deliver smoke in a continuous stream in proportion to the drawing effort provided by the smoker. Air may also be drawn into and mixed with the smoke stream via ventilation inlets provided at the periphery of the cigarette filter.

US 4,638,820 A discloses a smoking article comprising in combination a smoke producing material, air ventilation means for providing air to the smoking article and valve means operatively associated with said air ventilation means and adapted to move from a first position to a second position to substantially increase the concentration of air in the puff flow stream during the latter portion of each puff and then return to the first position.

### Description

This disclosure provides a flow delivery article such as, for example, a smoking article, a heat-not-burn product, an electronic-cigarette, or an aerosol/mist/vapour delivery article. The flow delivery article may include a tobacco industry article such as a cigarette or an e-cigarette.

The flow provided by the flow delivery article comprises a gaseous flow. The flow delivery article may deliver gaseous flow in the form of smoke, aerosol, air, vapour, mist or a mixture thereof. The gaseous flow may comprise particulate matter entrained within the flow.

In an embodiment, this disclosure provides a flow delivery article to deliver a gaseous flow to the mouth of a user, comprising a plurality of flow pathways, and a flow control component to repeatedly change the relative amount of flow through said pathways.

In an embodiment, this disclosure provides a flow delivery article to deliver a gaseous flow to the mouth of a user, comprising a flow control component to provide at least one flow during a draw on the flow delivery article, said at least one flow comprising a respective plurality of flow peaks, and a plurality of flow pathways, wherein the flow control component is arranged to repeatedly change the relative amount of flow through said pathways. The at least one flow may comprise a plurality of different flows, each flow comprising a respective plurality of flow peaks.

In an embodiment, this disclosure provides a flow delivery article to deliver a gaseous flow to the mouth of a user, comprising a flow control component to control the passage of flow to provide at least three consecutive flow pulses during a draw on the flow delivery article, and a plurality of flow pathways, wherein the flow control component is arranged to repeatedly change the relative amount of flow through said pathways. Each of said three consecutive flow pulses may be different.

As used herein the term "flow delivery article" includes products which deliver a gaseous flow, for example smoking articles, heat-not-burn products, electronic-cigarettes, and aerosol/mist/vapour delivery devices. Preferably, the flow delivery article comprises a tobacco industry article such as a cigarette or e-cigarette. The flow provided by the flow delivery article comprises a gaseous flow.

The flow delivery article may deliver flow in the form of one or more of smoke, aerosol, air, vapour or mist. In some examples the plurality of flow sources may include a source of smoke having a first flavour, and a source of smoke having a second flavour.

In various embodiments, the flow control component comprises a movable member, wherein movement of the movable member changes the relative amount of flow through different flow pathways. The movable member may be arranged so that received flow causes the movable member to move.

The movable member may comprise a rotary member configured so that rotation of the rotatable member changes the relative amount of flow through said flow pathways.

The rotary member may comprise a flow-driven rotary member comprising one or more flow-receiving portions configured to cause rotation of the rotary member in response to receiving flow.

In an embodiment, the flow control component comprises a progressive cavity device.

In an embodiment, the movable member comprises a first flow-receiving portion to cause rotation of the rotary member in response to receiving a first flow, and a second flow-receiving portion to cause rotation of the rotary member in response to receiving a second flow.

The first flow-receiving portion may comprise a helical element and the second flow-receiving portion may comprise a helical element.

The first flow-receiving portion may comprise one or more paddles and the second flow-receiving portion may comprise one or more paddles.

In various embodiments, the flow delivery article comprises a smoking article.

The smoking article may comprise a tobacco rod and a filter, wherein the filter comprises the flow control component.

The flow control component may be positioned between the filter and the tobacco rod. Alternatively, the flow control component may be positioned within the filter.

In various embodiments, the flow control component is located at the mouth end of the smoking article.

In an embodiment, the movable member comprises a fan arranged so that received flow causes the fan to rotate. The smoking article may further comprise a support member to support the movable member in the flow.

The movable member may comprise a rotatable opening configured to permit flow through the first flow pathway in a first rotational position and through the second flow pathway in a second rotational position.

The movable member may comprise a valve.

The flow control component may be configured to provide a flow comprising a train of pulses.

The flow delivery article may comprise a flavouring component to flavour at least some of said pulses.

The flow control component may be configured to generate vibration to provide tactile stimulation to a user.

Various embodiments provide a flow delivery article in the form of a smoking article, the smoking article comprising any of the various flow control components of this disclosure.

As used herein, the term "smoking article" includes smokeable products such as cigarettes, cigars, cigarillos and pipes, whether based on tobacco, tobacco derivatives, expanded tobacco, reconstituted tobacco or tobacco substitutes and also heat-not-burn products.

In some embodiments, a smoking article comprise a combustion-based smoking article such as a cigarette. Alternatively, in other embodiments, a smoking article comprises a non-combustion-based article such as an electronic cigarette, or other non-combustion-based device which is smoked in use.

In an embodiment, this disclosure provides a smoking article, comprising a flow control component to provide at least one flow during a draw on the smoking article, said at least one flow comprising a respective plurality of flow peaks. The at least one flow may comprise a plurality of different flows, each flow comprising a respective plurality of flow peaks.

In an embodiment, this disclosure provides a smoking article comprising a flow control component to control the passage of at least one flow to provide a plurality of flow peaks. The at least one flow may comprise at least one of a smoke flow, an air flow, a flavoured flow and a mixed smoke/air/flavoured flow.

In an embodiment, this disclosure provides a smoking article comprising a flow control component configured to cause a plurality of flow changes during a draw on the smoking article, thereby to provide a time-varying flow.

Each flow change may comprise changing the relative amount of flow through different flow pathways.

In an embodiment, this disclosure provides a smoking article comprising a flow control component configured to provide at least three consecutive flow pulses during a draw on the smoking article.

The disclosure also provides a smoking article comprising a flow control component to control the passage of flow to provide at least three consecutive flow pulses. Each of said three consecutive flow pulses may be different.

The flow control component may be configured to provide a repeating plurality of said three consecutive flow pulses.

As used herein, the terms "flavour" and "flavourant" refer to materials which, where local regulations permit, may be used to create a desired taste or aroma in a product. They may include extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Dramboui, bourbon, scotch, whiskey, spearmint, peppermint, lavender, cardamon, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour masking agents, bitterness receptor site blockers, receptor site enhancers, sweeteners (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

This disclosure also provides a filter for a smoking article comprising any of the various flow control components of this disclosure.

In various embodiments, the flow control component is configured to a pulse frequency in the range 50 Hz to 150 Hz, such as between 60 Hz and 100 Hz, or approximately 70 Hz, or approximately 80 Hz may be generated. In embodiments, a pulse frequency in the range 30 Hz to 1000 Hz, such as between 50 Hz and 200 Hz, or such as between 60 Hz and 70Hz, may be generated.

In order that the invention of this disclosure may be more fully understood, embodiments thereof will now be described by way of example only, with reference to the accompanying drawings, in which:
Figure 1 is an axial section through an exemplary smoking article in accordance with the disclosure.
Figure 2 is an axial section showing the flow control component of the exemplary smoking article in more detail;
Figure 3 is a perspective view of the smoking article with a part thereof removed, showing the exemplary flow control component.
Figure 4 is a perspective view of the exemplary flow control component with housing removed, showing the rotor assembly in a first position;
Figure 4a shows a stator part of the flow control component;
Figure 5 is a perspective view of the exemplary flow control component with housing removed; showing the rotor assembly in a second position;
Figure 6 schematically illustrates an example of pulsed flow;
Figure 7 illustrates exemplary variations of the rotary disk of the rotor assembly;
Figure 8 is a perspective view of a cigarette comprising a flow control component positioned between a tobacco rod and a filter plug;
Figure 9a illustrates the smoke channel part of the flow control component;
Figure 9b illustrates the air channel part of the flow control component;
Figure 10 illustrates the paddlewheel of the flow control component;
Figure 11a is a perspective view of a cigarette comprising a flow control component positioned at the mouth end, adjacent to a filter plug;
Figure 11b illustrates the flow control component of Figure 11a in more detail;
Figure 12a shows the rotatable element with a part thereof removed, showing the internal helical screw;
Figure 12b shows the supporting element;
Figures 13a - c show the rotatable element in different positions within the flow control component;
Figure 14a is a perspective view of a cigarette comprising a flow control component positioned at the mouth end, adjacent to a filter plug;
Figure 14b illustrates the flow control component of Figure 14a in more detail;
Figure 14c shows part of the flow control component;
Figure 14d is a perspective view of the helical rotor of the flow control component;
Figure 14e shows the mouth end part of the flow control component;
Figure 14f shows the flow channel housing of the flow control component
Figures 15a - c are diagrams showing the rotor in different positions within the flow control component;
Figures 16a - d are diagrams showing different arrangements of flow control components;
Figure 17 illustrates another exemplary flow delivery article in accordance with the disclosure;
Figure 18 separately illustrates the smoke and air flow components of an exemplary pulsed flow;
Figure 19 illustrates another exemplary flow delivery article in accordance with the disclosure;
Figure 20 separately illustrates the smoke and air flow components of an exemplary pulsed flow;
Figure 21 illustrates another exemplary flow delivery article in accordance with the disclosure;
Figure 22 illustrates another exemplary flow control component in accordance with the disclosure;
Figure 23 illustrates a variation of the exemplary flow control component of Figure 13.

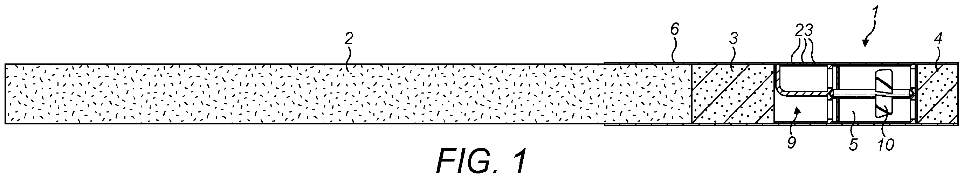

For purposes of illustration, but not limitation, Figure 1 shows a sectional view of a flow delivery article in the form of a cigarette 1. As shown, cigarette 1 comprises a smoke generating component in the form of a tobacco rod 2, and first and second filter plug components 3, 4. Cigarette 1 further comprises a flow control component 5 which is positioned between the two filter plug components 3, 4. As shown, the tobacco rod 2, filter components 3, 4 and flow control component 5 are longitudinally aligned and wrapped with a tipping paper 6 to hold them together.

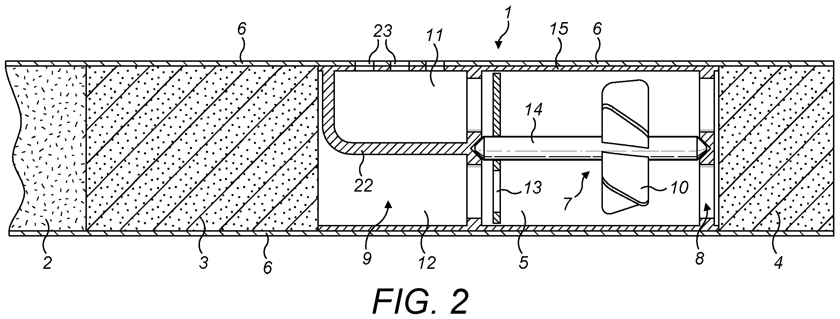

Referring to Figures 2-4, flow control component 5 includes a movable member in the form of rotor assembly 7, which is rotatably mounted between first and second stator parts 8, 9. As shown, rotor assembly 7 includes a fan 10, which is driven to rotate by air and smoke flow drawn by the smoker from air and smoke channels 11, 12 formed in the second stator part 9. The rotor assembly includes an opening 13 which alternately aligns with the air channel 11 and the smoke channel 12 during rotation so that a pulsed flow comprising alternating air and smoke pulses is generated.

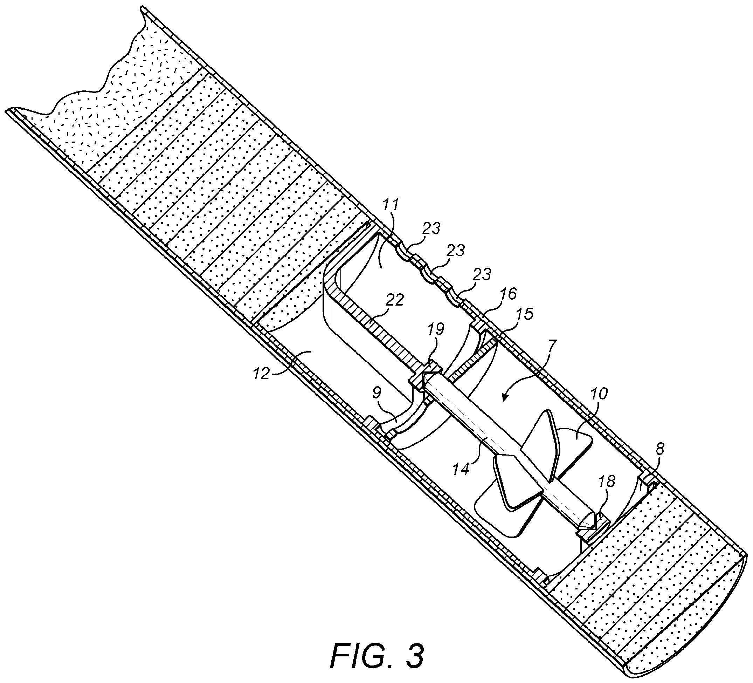

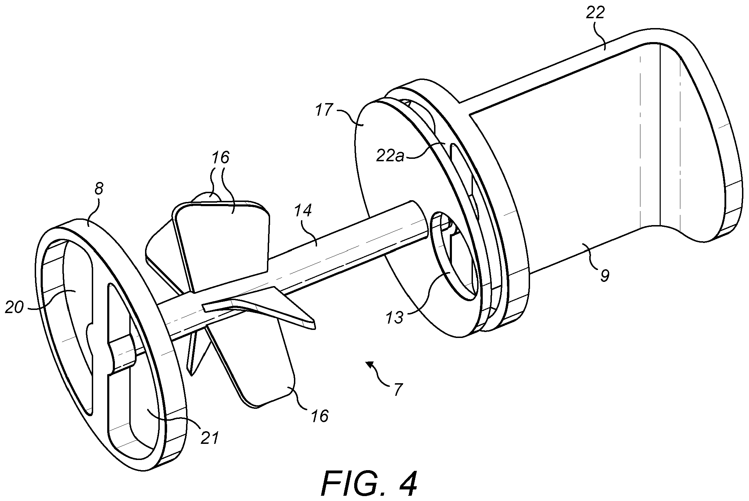

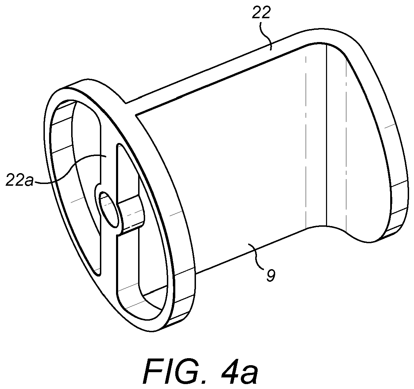

Turning now to a more detailed description of the flow control component 5, as shown in Figures 2 and 3, the rotor assembly 7 includes a shaft 14, which is rotatably mounted to the stator parts 8, 9. The rotor assembly 7 and stator parts 8, 9 may be contained in an optional housing comprising a solid cylindrical sleeve 15, shown in Figures 2 and 3. Figure 4 shows a perspective view with the sleeve 15 removed. Figure 4a shows the stator part 9 in isolation.

As shown in Figure 4, blades 16 are attached to and extend from the shaft 14 to form the fan 10. A rotary disk 17 is attached to the shaft 14 at a position longitudinally displaced from the fan 10. The rotary disk 17 includes the opening 13, which rotates in use with the rest of the rotor assembly 7.

As shown in Figure 3, one end of the shaft 14 is rotatably mounted in a hub region 18 of the first stator part 8. The opposite end of the shaft is rotatably mounted in a hub region 19 of the second stator part 9. Bearings may also be provided at the hub regions 18, 19 to facilitate rotation of the rotor assembly 7.

As shown in Figure 4, the first stator part 8 is disk-shaped and includes two openings 20, 21 to permit flow passage through the stator part 8. Referring again to Figure 3, the second stator part 9 is formed to define separate channels 11, 12 for air and smoke. As shown, the air and smoke channels 11, 12 are separated by an L-shaped barrier 22.

As shown in Figures 2 and 3, air inlets 23 are formed at the periphery of the cigarette 1 to permit air to enter the air channel 11. The air inlets comprise a plurality of holes through one side of the housing 15. The air inlets further comprise holes formed in the tipping paper 6 in alignment with the holes in the housing 15. In some implementations, the housing 15 may be omitted. In some implementations, the tipping paper may be formed from air permeable material so that air permeates through the paper.

When the opening 13 in the rotary disk 17 is aligned with the air channel 11 in the position shown in Figure 4, a flow pathway for air is defined from air inlets 23 in the periphery of the filter into and through the air channel 11 and through opening 13 so as to reach the fan 10. Air can thus be drawn through the air flow path by the smoker so as to contact and drive rotation of the fan 10. Air passing by the fan 10 passes through the openings 20, 21 in the first stator part, then through the mouthend filter component 4 and into the smoker's mouth.

When the opening 13 is in the position of Figure 4, smoke passage through the smoke channel 12 is obstructed by the rotary disk 17. Also, smoke is prevented from passing into the air channel 11 by the barrier 22. As a result, in this position, the flow drawn from the cigarette 1 is substantially or entirely air. In some implementations it may be desirable to mix an amount of smoke with the air flow, and in this case a longitudinal gap between the rotary disk 17 and the second stator part 9 may be provided to allow some smoke to flow from the smoke channel 12 along a circuitous path to the opening 13. However, in other implementations, the longitudinal gap between the rotary disk 17 and the second stator part can be made sufficiently small so that only air is drawn from the cigarette in the position of Figure 4.

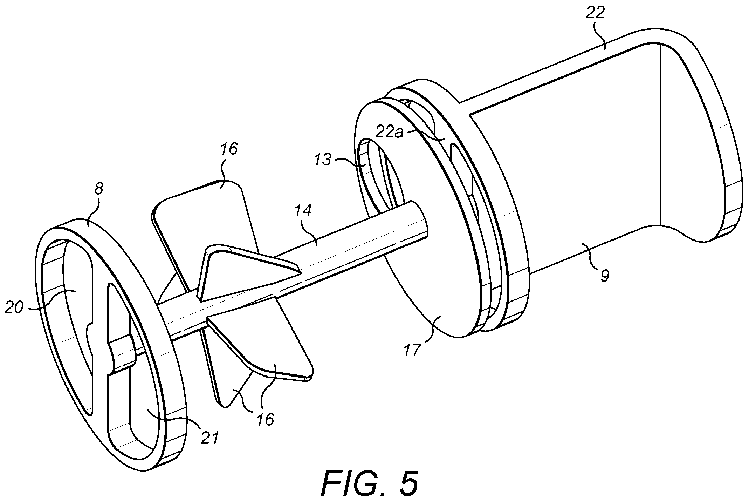

When the opening 13 is aligned with the smoke channel 12 as shown in Figure 5, a flow path for smoke is defined from the tobacco rod 2 through the filter component 3, through smoke channel 12 and through opening 13. Smoke can thus be drawn from the tobacco rod 2 and through the smoke flow path by the smoker so as to contact and drive rotation of the fan 10. Smoke passing by the fan 10 passes through the openings 20, 21 in the first stator part, then through the mouth end filter component 4 and into the smoker's mouth.

When the opening 13 is in the position of Figure 5, air passage through the air channel 11 is obstructed by the rotary disk 17. Also, air is prevented from passing into the smoke channel 12 by the barrier 22. As a result, in this position, the flow drawn from the cigarette 1 is substantially or entirely smoke. In some implementations an amount of air may flow from the air channel 11 along a circuitous path to the opening 13, via a longitudinal gap between the rotary disk 17 and the second stator part 9, so as mix with the smoke flow drawn from the some channel 12. In other implementations, the longitudinal gap between the rotary disk 17 and the second stator part can be made sufficiently small so that only smoke is drawn from the cigarette in the position of Figure 5.

In use, the smoker draws on the cigarette and the resulting flow drives the fan 10, thereby causing rotation of the opening 13. Flow in the form of smoke or air reaches the fan and drives rotation whether the opening 13 is aligned with the air channel 11 as in Figure 4 or with the smoke channel 12 as in Figure 5. In the intermediate position where the opening 13 straddles an end region 22a of barrier 22, part of the opening 13 is aligned with the air channel and part is aligned with the smoke channel. In this position, there is a flow path for smoke and also a flow path for air through the opening 13, so that both air and smoke reach and turn the fan 10 before passing into the smoker's mouth.

Thus, as the smoker draws on the cigarette, the rotating opening alternately aligns with the air and smoke channels 11, 12. In this way, the flow control component acts as a valve which repeatedly switches flow between the smoke and air pathways. Thus, the smoker is provided with a pulsed flow comprising alternating smoke and air pulses.

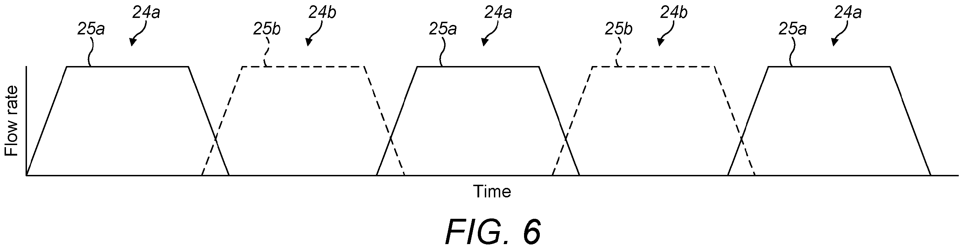

Figure 6 schematically illustrates an example of pulsed flow during a draw period according to an embodiment. As shown, the total flow includes a smoke flow component, comprising a plurality of smoke pulses 24a, and an air flow component comprising a plurality of air pulses 24b. Each smoke pulse 24a is followed by an air pulse 24b, so that smoke and air pulses 24a, 24b alternate in the pulsed flow. Thus, a train of alternating air/smoke pulses is provided.

Although for example the smoke flow rate may, in some implementations, fall to zero at certain times as shown in Figure 6, this is not intended to be a limiting feature of pulsed smoke flow. In some implementations, each air pulse 24b may substantially comprise air, but may also include an amount of smoke, e.g: drawn along a circuitous path from the smoke channel 12, as described above with reference to Figure 4. Similarly, in some implementations, each smoke pulse may include some air drawn along a circuitous path from the air channel 11, as described above with reference to Figure 5.

As shown in Figure 6, as a smoke pulse builds up, the previous air pulse diminishes and vice-versa, so that the aggregate flow (ie: smoke and air pulses combined) may in some implementations remain constant or substantially constant as the opening 13 rotates. As shown in Figure 4, in some rotational positions, the opening 13 may be partially blocked by the end region of the barrier 22a, so as to cause the aggregate flow to fall and then rise again during rotation. The width of the end region of the barrier 22a may be selected to obtain a desired degree of variation in the aggregate flow.

As shown in Figure 6, each smoke pulse 24a defines a flow peak 25a of the smoke flow. Similarly, each air pulse 24b defines a flow peak 25b of the air flow. The air flow peaks 25b are temporally offset from one another. Similarly, the smoke flow peaks 25a are temporally offset from one another, so as to provide a pulsed smoke stream.

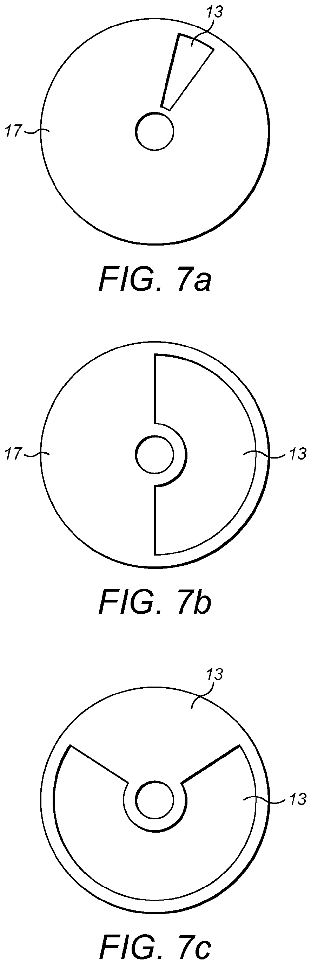

Many variations of the flow control component 5 are possible. For example, although the opening 13 shown in Figure 4 is circular, alternatively the opening could be a different size or shape. Also, rather than a single opening, multiple openings could alternatively be provided. By altering the size, shape and/or number of openings, the characteristics of the air and smoke pulses of Figure 6 may be varied. For example, the opening 13 may be altered so that the pulse amplitude, pulse duration and/or pulse shape is varied. Figure 7a, 7b and 7c illustrate three exemplary variations of the opening 13.

In some examples, the opening shape shown in Figure 7a may be sufficiently narrow in width so the entire cross sectional area of opening 13 may be obstructed by the end region of barrier 22 in some rotational positions. Although this particular shape of opening may result in a brief period in which no flow, or little flow, reaches the fan 10, the momentum of the moving fan 10 will nonetheless tend to cause the opening 13 to continue rotate until it once again aligns with a flow channel 11, 12, thereby driving the fan again. In some implementation, the opening shape of Figure 7a may therefore have the effect of introducing a period after each smoke and air pulse in which no flow is provided at the output of the cigarette. Such "no flow" gaps between neighbouring smoke and air pulses provides a further variation to smoking experience, which may be desirable to consumers.

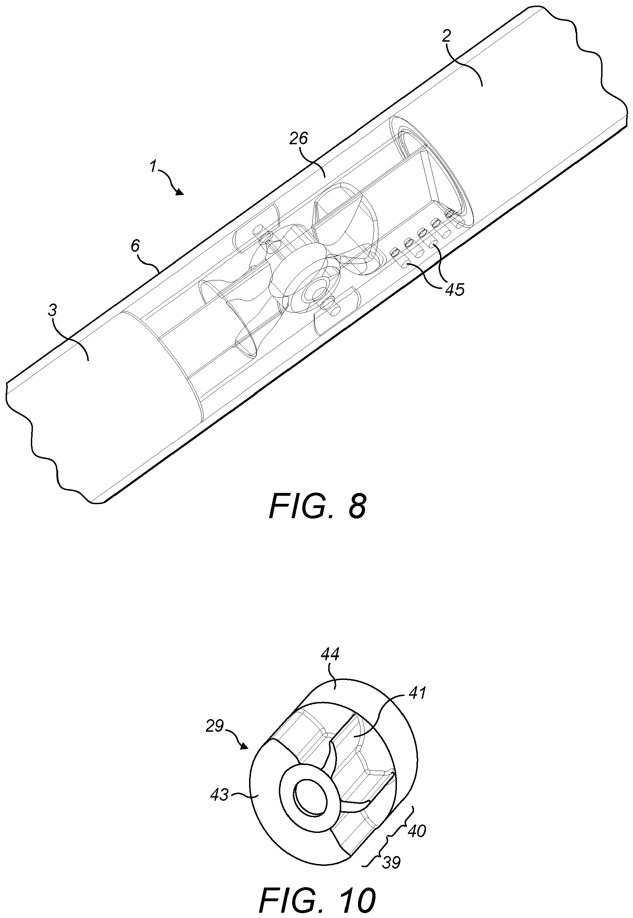

Figure 8 shows a sectional view of a cigarette 1 comprising a flow control component 26 in place between tobacco rod 2 and filter plug 3. As shown, the tobacco rod 2, filter plug 3, and flow control component 26 are longitudinally aligned and wrapped with a tipping paper 6 to hold them together.

Component 26 comprises first and second parts 27, 28, wherein the first part 27 is a smoke channel part 27, and the second part 28 is an air channel part 28. Component 26 also comprises a rotary member 29 in the form of a cylindrical turbine or paddlewheel 29 configured to rotate relative to the parts 27, 28. The paddlewheel 29 is rotatably mounted within the two channel parts 27, 28.

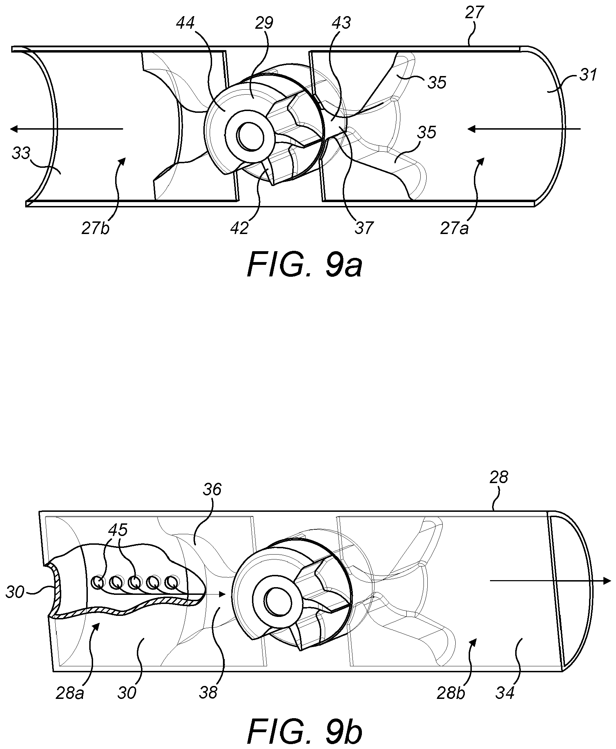

Figure 9a shows a perspective view of the smoke channel part 27 with the paddlewheel 29 in situ. Figure 9b shows a perspective view of the air channel part 28 with the paddlewheel 29 in situ. Figure 10 shows the paddlewheel 29 in isolation.

As shown in Figures 9a and 9b, the outer surface of each of the two channel parts 27, 28 is in the form of a half cylinder, such that when combined, the two channel parts 27, 28 form the cylindrical flow control component 26. In some embodiments, the two channel parts 27, 28 may be contained in a housing comprising a solid cylindrical sleeve. At least one of the channel parts, in this case the second channel part 28, comprises a barrier 30. As shown in Figure 8, when combined to form the component 26, the first and second channel parts 27, 28 define separate channels for smoke flow and air flow, wherein the smoke flow and air flow channels are separated by the barrier 30. The barrier 30 is shaped to accommodate the paddlewheel 29. There is substantially no leakage of flow from one of the channel parts to the other.

Each of the two channel parts 27, 28 has a tobacco rod end 27a, 28a and a mouth end 27b, 28b. An inlet section 31, 32 comprising a cavity for receiving flow into the component 26, is located between the tobacco rod end of the channel part 27a, 28a and the paddlewheel 29. An outlet section 33, 34 is located between the paddlewheel 29 and the mouth end of the channel part 27, 28.

To increase the torque applied by the air flow and smoke flow to the paddlewheel 29, the inner surface 35, 36 of the two channel parts 27, 28 is shaped to direct air or smoke substantially towards and around the peripheral surface of the paddlewheel 29. There may be a gap between the channel part 27, 28 and the paddlewheel 29, which may allow air or smoke to be drawn around the paddlewheel 29. The inner surface 35, 36 of the channel parts 27, 28 also comprises a constricted region 37, 38 which may lead to a venturi effect, increasing the velocity of the air or smoke flow.

The paddlewheel 29 is shown in detail in Figure 10. The paddlewheel 29 is generally cylindrical shape, having a first end 39 and a second end 40, which are fixed to one another. The paddlewheel 39 is rotatably mounted within the two channel parts 27, 28 such that the first end 39 of the paddlewheel 29 is positioned within the smoke channel part 27, and the second end 40 of the paddlewheel 29 is positioned within the air channel part 28.

The first and second ends 39, 40 of the paddlewheel 29 are axially aligned, and in the embodiment shown, are identical apart from being formed to be rotated 180° with respect to one another.

The first and second ends 39, 40 each include a respective paddle region having a plurality of paddles 41, 42. As shown in Figure 9a, 9b and 10, the paddle region of the first end is formed to be rotated 180° with respect to the paddle region of the second end.

As shown, the end 39 also comprises a solid half-cylindrical portion 43 which is not formed into paddles 41. The radius of the portion 43 approximately matches that of the arc defined by the tips of the paddles 41.

Similarly, the end 40 also comprises a solid half-cylindrical portion which is not formed into paddles 42. The radius of the portion 44 approximately matches that of the arc defined by the tips of the paddles 42.

As described in more detail below, the portions 43, 44 act as flow-blocking portions which periodically block flow through respective flow channels as the paddlewheel 29 rotates. When the flow-blocking portion 43 of the first end 39 blocks the smoke channel, air flow in the air channel drives rotation of the paddle region of the second end 40 to turn the paddlewheel 29. Similarly, when the portion 44 of the second end 40 blocks the air channel, smoke flow in the smoke channel drives rotation of the paddle region of the first end 39 to turn the paddlewheel 29. In this way, the paddlewheel 29 is continually driven to rotate by flow drawn through the smoke and air channels.

Although Figures 8, 9a, 9b and 10 show the regions 43, 44 to each have a smooth curved peripheral surface, in some embodiments, each region 43, 44 may have a plurality circumferentially spaced shallow ridges defined in its peripheral surface.

The smoke channel part 27 is shown in detail in Figure 9a. When the paddles 41 of the paddlewheel 29 are aligned with the constricted region 37 of the smoke channel part 27, a flow pathway for smoke is defined from the tobacco rod 2, into the smoke channel inlet section 31, so as to reach the paddles 41 of the first end 39 of paddlewheel 29. In use, smoke can thus be drawn through the smoke flow path by the smoker so as to contact the paddles 41 and drive rotation of the paddlewheel 29. In this way, the paddlewheel 29 is driven to rotate by smoke flow drawn by the user. Smoke passing by the paddlewheel 29 passes through the smoke channel outlet section 33 and into the smoker's mouth.

When the flow-blocking portion 43 of the paddlewheel 29 is aligned with the constricted region 37 of the smoke channel part 27, flow of smoke is prevented from passing by the paddlewheel 29. In this case, no smoke may be drawn into the mouth of the user.

The air channel part 28 is shown in detail in Figure 9b. The tobacco rod end 28a of the air channel part 28 is sealed by barrier 30 so that in use smoke from the tobacco rod 2 cannot be drawn into the air channel part 28. Air inlets 45 are formed at the periphery of the smoking article 1 so that in use air may be drawn into the air channel inlet 32. The air inlets 45 comprise a plurality of holes through the side of the air channel part 28. The air inlets further comprise holes formed in the tipping paper 6 in alignment with the holes in the air channel part 28. In some implementations, the tipping paper 6 may be formed from unperforated air permeable material so that air permeates through the paper.

When the paddles 42 of the paddlewheel 29 are aligned with the constricted region 38 of the air channel part 28, a flow pathway for air is defined from air inlets 45 into the air channel inlet 32, so as to reach the second end 40 of the paddlewheel 29. Air can thus be drawn through the air flow path by the smoker so as to contact the paddles 42 and drive rotation of the paddlewheel 29. In this way, the paddlewheel 29 is driven to rotate by air flow drawn by the user. Air passing by the paddlewheel 29 passes through the air channel outlet section 34, and into the user's mouth.

When the flow-blocking portion 44 of the paddlewheel 29 is aligned with the constricted region 38 of the air channel part 28, flow of air is prevented from passing by the paddlewheel 29 and thus no air may be drawn into the mouth of the user.

At any given moment, some or all of the paddles 41, 42 of the first or second ends 39, 40 of the paddlewheel 29 are aligned with the corresponding constricted region 37, 38 and are available to receive flow drawn by the user. Thus in use, as the user draws on the cigarette the resulting flow in the form of smoke or air drives rotation of the paddlewheel 29.

When the paddles 41 are aligned with the constricted region 37 of the smoke channel part 27, the flow-blocking portion 44 is aligned with the constricted region 38 of the air channel part 28. Air passage through the air channel part 28 is thereby prevented. Also, air is prevented from passing into the smoke channel part 27 by the barrier 30. As a result, in this position, the flow drawn from the cigarette 1 is substantially or entirely smoke. This smoke flow drives rotation of the paddlewheel 29 which causes the flow-blocking portion 43 to become aligned with the constricted region 37 of the smoke channel part 27, thus preventing further flow of smoke. Smoke is also prevented from passing into the air channel part 28 by the barrier 30. As a result, in this position, substantially no smoke may be drawn from the cigarette 1.

Rotation of the paddlewheel 29 causes the paddles 42 to become aligned with the constricted region 38 of the air channel part 28. Thus air may be drawn through the air channel part 28. This air flow drives further rotation of the paddlewheel 29.

Thus, as the smoker draws on the cigarette, the resulting rotation of the paddlewheel 29 causes the paddles 41, 42 to alternately align with the air and smoke channels 27, 28. In this way, the flow control component acts as a valve which repeatedly switches flow between the smoke and air pathways. Thus, the smoker is provided with a pulsed flow comprising alternating smoke and air pulses.

Many variations of the flow control component 26 are possible. In some implementations there may be a gap between the paddlewheel 29 and one or both of the parts 27, 28. In addition or alternatively, the flow-blocking portions 43, 44 may comprise a number of shallow ridges. In these implementations, there may be passage of some air or smoke flow even when the paddles 41, 42 are not aligned with the corresponding air or smoke channels 27, 28. This leakage of flow may improve the rotational properties of the paddlewheel 29.

In the embodiment shown, the barrier 30 prevents any mixing of the air and smoke flows until the flows pass out of the outlet sections 33, 34. In other embodiments, however, the outlet sections 33, 34 may be arranged to modify the pulsed air and smoke flows. For example, the outlet sections may be arranged so that the air and smoke flows are combined to form a single pulsed flow within the flow control component 26. In other embodiments, the outlet sections 33, 34 may be arranged to deliver a flavourant to the pulsed air and smoke flows.

In some implementations the flow control component may comprise three channels, for example, for the pulsed delivery of smoke, air, and flavourant. In these implementations, the component may comprise a separate smoke channel part, air channel part, and flavourant channel part. In addition, the paddlewheel may be separated into thirds along its length, wherein each third may be shaped such that a third of its circumference comprises paddles, and two thirds comprises a flow-blocking portion. In use, the flow of smoke, air, or flavourant drawn by the user drives rotation of the paddlewheel causing pulsed flow of smoke, air, or flavourant.

Many variations of the flow control component 26 are possible. In the embodiment shown in Figures 8 to 10, the axis of rotation of the paddlewheel 29 is aligned perpendicularly to the longitudinal axis of the smoking article 1. In other embodiments, however, the paddlewheel 29 may be aligned at an oblique angle to the longitudinal axis of the smoking article 1. In these embodiments, to increase the torque applied by the smoke flow to the paddlewheel 29, the paddles 41, 42 may have a helical configuration around the circumferential surface of the paddlewheel 29. In these embodiments, the paddlewheel may be rotatably mounted by means of a tubular cylindrical hub. The hub may comprise a further flow path, which may be arranged to deliver a pulse flow.

In some embodiments, instead of a cylindrical paddlewheel, the movable member may have an approximately spherical or ovoid shape. A spherical shape may advantageously be employed to optimise the volume of flow which is used to drive rotation, especially when employed in combination with a substantially cylindrical conduit, so as to optimise interaction between the smoke and the movable member.

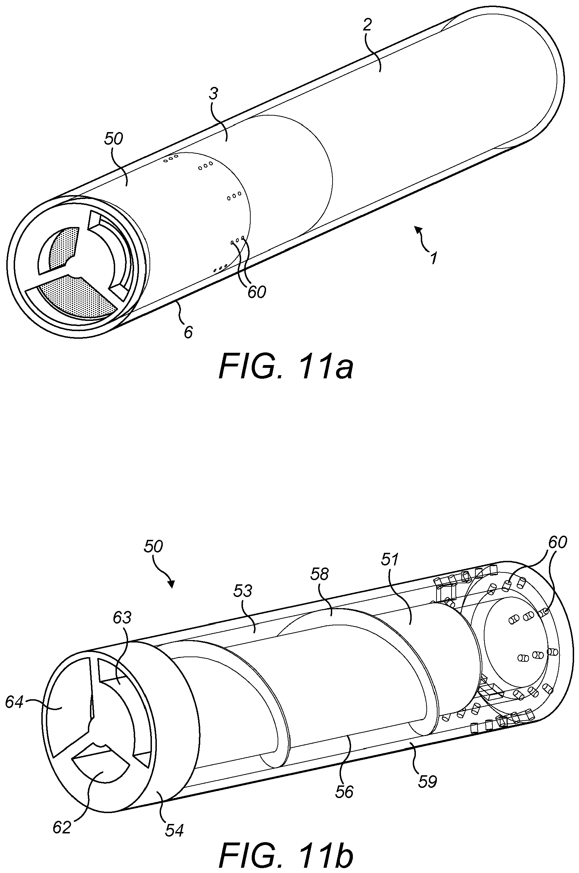

Figure 11a shows another flow control component 50 in place at the mouth end of a cigarette 1. Cigarette 1 further comprises a tobacco rod 2 and a filter plug 3. As shown, the tobacco rod 2, filter plug 3, and flow control component 50 are longitudinally aligned and wrapped with a tipping paper 6 to hold them together.

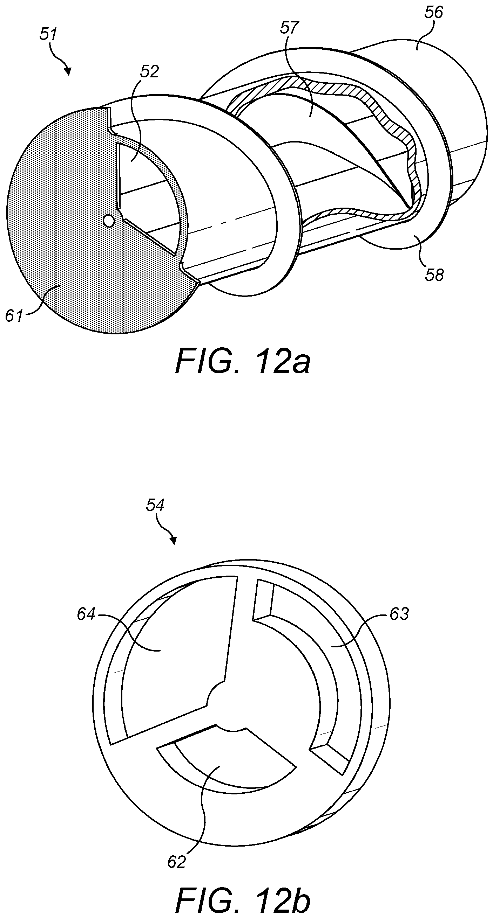

The flow control component 50 is shown in more detail in Figure 11b. The component 50 has the form of an axial turbine comprising a rotatable element 51, which is shown in isolation in Figure 12a. The rotatable element 51 comprises a smoke flow channel 52 and an air flow channel 53. In use, the rotatable element 51 is driven to rotate by means of air and smoke flow drawn by the user. The rotatable element 51 rotates relative to a supporting element 54, which is shown in isolation in Figure 12b. The supporting element 54 comprises a plurality of openings 62, 63, 64. As the rotatable element 51 rotates, the air and smoke channels 52, 53 sequentially align with the openings, so that a pulsed flow comprising air and smoke pulses is generated.

Figure 12a shows the rotatable element 51. The rotatable element 51 comprises a cylindrical tube 56. The core of the tube 56 comprises a helical screw 57, which defines a helical smoke flow channel 52 through the tube 56. In use, smoke flow may be drawn from the tobacco rod 2 through the filter plug 3 and through the smoke flow channel 52. The smoke flow drives rotation of the helical screw 57 so as to cause rotation of the rotatable element 51.

The rotatable element 51 further comprises one or more helical vanes 58 spiralling along the outer circumferential surface. As shown in Figure 11b, the rotatable element 51 is contained in and rotates relative to a housing comprising a solid cylindrical sleeve 59. The air flow channel is formed between the rotatable element 51 and the sleeve 59.

Air inlets 60 are formed at the periphery of the smoking article 1 so that in use air may be drawn into the air flow channel 53. The air inlets 60 comprise a plurality of holes through the side of the sleeve 59. The air inlets 60 further comprise holes formed in the tipping paper 6 in alignment with the holes in the sleeve 59. In some implementations, the tipping paper may be formed from unperforated air permeable material so that air permeates through the paper.

In use, air may be drawn through the air inlets 60 and along the cavity formed between the rotatable element 51 and the sleeve 59. The smoke flow thus acts on the helical vanes 58 so as to cause rotation of the rotatable element 51.

As shown in Figure 12a, the mouth end of the rotatable element comprises an end cap 61 in the form of a 240° segment of a flat disk. As shown in Figure 11b, when the rotatable element 51 is in place within the sleeve 59, two thirds of the surface area of the smoke channel 52 and two thirds of the surface area of the air channel 53 is closed by the end cap 61.

As shown in Figure 11b, the component 50 further comprises at the mouth end a supporting element 54. Figure 12b shows the supporting element 54 in isolation. The rotatable element 51 is rotatably supported at the mouth end by the supporting element 54.

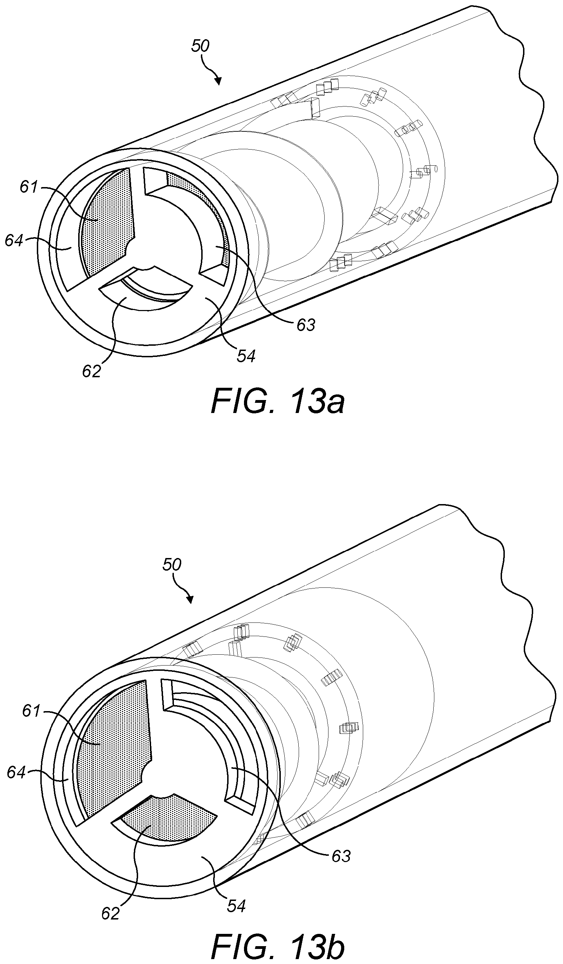

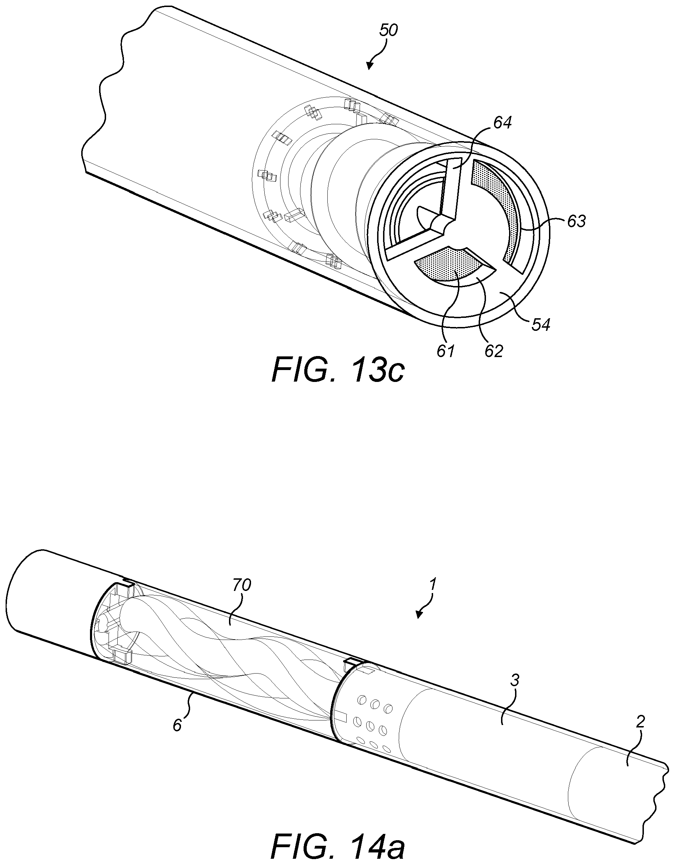

The fixed supporting element 54 comprises first, second, and third openings 62, 63, 64. The first opening 62 comprises the smoke flow opening and provides a passage through which smoke may be drawn from the smoke flow channel 52 into the mouth of the user. Thus, as shown in Figure 13a, when the rotatable element 51 is positioned such that the end cap 61 does not block the first opening 62, smoke may be drawn into the mouth of the user. Smoke flow thus acts on the helical screw 57 to drive rotation of the rotatable element 51. Rotation of the rotatable element 51 causes the end cap 61 to align with and block the first opening 62, thus preventing the flow of smoke through this opening. Simultaneously, as the rotatable element 51 rotates, the end cap 61 moves out of alignment with the second opening 63, as shown in Figure 13b. As a result, air may be drawn from the air flow channel 53, through the second opening 63, and into the mouth of the user. This air flow acts on the helical vanes 58 to drive further rotation of the rotatable element 51. Rotation of the rotatable element 51 causes the end cap 61 to rotate into a position in which the first and second openings 62, 63 are blocked, and in which a flow path is defined through the third opening 64, as shown in Figure 13c. The third opening 64 provides a passage for the simultaneous flow of smoke from the smoke flow channel 52 and air from the air flow channel 53 into the mouth of the user. Flow of smoke and air through the third opening 64 drives further rotation of the rotatable element. Thus, as the user draws on the cigarette, the resulting rotation of the rotatable element causes the smoke and/or air channels 52, 53 to alternately align with the openings 62, 63, 64. In this way, the flow control component acts as a valve which repeatedly switches flow between the smoke, air, and smoke and air pathways.

In other embodiments, the flow control component may comprise a third flow path, for example comprising a flavourant. This may be achieved, for example, by separating the core of the cylindrical tube 56 into an inner core comprising a first helical screw and an outer core comprising a second helical screw. By modification of the openings 62, 63, 64 in the supporting element 54, flow pluses may be sequentially delivered from each of the flow paths.

In other embodiments, pulsed flow may be provided in other ways. For example, the flow control component may comprise a second cylindrical sleeve, comprising air inlets, which is attached to the rotatable element and rotates within the first cylindrical sleeve. In these embodiments, air may be drawn into the air flow channel as the air inlets in the two cylindrical sleeves align. When the air inlets are not aligned, there is no flow of air. In these embodiments, a constant flow of smoke may be drawn. In addition, the openings in the supporting element 54 may have any suitable shape.

In some embodiments, the rotatable element 51 may be eccentrically weighted to provide vibration of the component in combination with a pulsed flow. For example, the rotatable element 51 may be eccentrically weighted by means of an unbalancing mass incorporated, for example, within the helical vane 58. In addition, or as an alternative, the axis about which the rotatable element 51 rotates may be off-centred so that the element rotates eccentrically. In general, in such embodiments, the rotatable element 51 may be arranged so that the centre of mass of the rotatable element 51 does not lie on the axis of rotation.

Figure 14a shows another flow control component 70 in place adjacent to a filter plug section 3 at the mouth end of a smoking article 1. The tobacco rod 2, filter plug 3 and flow control component 70 are longitudinally aligned and wrapped with a tipping paper 6 to hold them together. The flow control component 70 comprises a progressive cavity device.

Progressive cavity devices (such as the eccentric screw pump, also known as a cavity pump or Moineau pump) are well known *per se* and will not be described in detail here. Briefly, a progressive cavity device comprises a rotary element configured to rotate to cause one or more cavities to move through the device, thereby to transfer flow.

It is known *per se* to provide a progressive cavity device as a pump device to transfer fluid or as a motor, for example in oilfield applications. In contrast, the progressive cavity device 70 of Figure 14a is a turbine driven by the flow drawn from the smoking article 1.

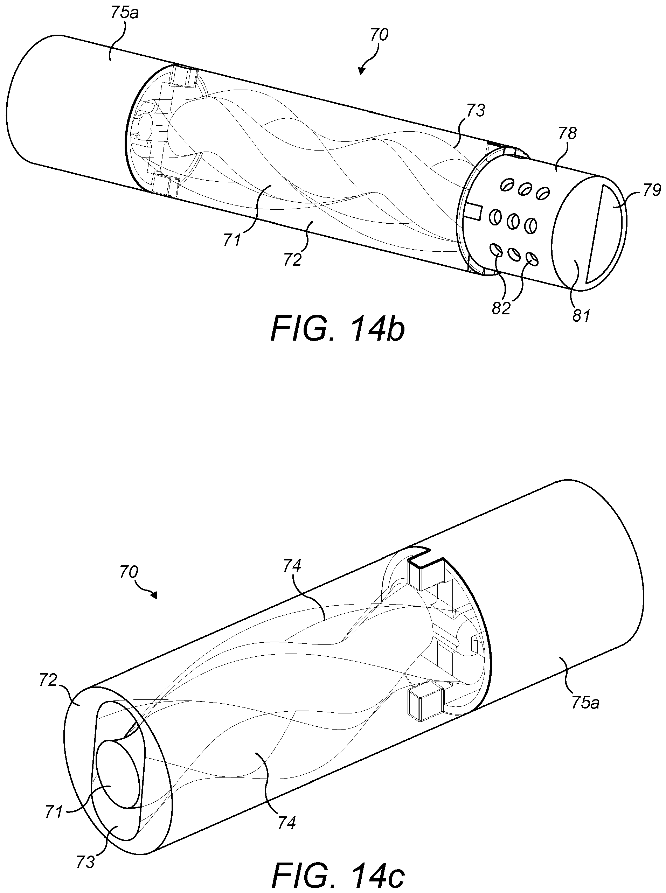

Turning to a more detailed description of the flow control component 70, as shown in Figure 14b, the progressive cavity assembly comprises a helical rotor 71 and a stator housing 72. The stator housing 72 has an inner surface 73 defining a smoke conduit.

Figure 14c is a view of a part of the progressive cavity assembly. In the embodiment shown, the rotor 71 is circular in cross-section. The inner surface 73 of the stator housing 72 is shaped along its length to form a double lobed helix, and a number of fixed size cavities 74 are thus formed within the housing 72 between the rotor 71 and the internal surface 73 of the housing at any particular rotational position of the rotor.

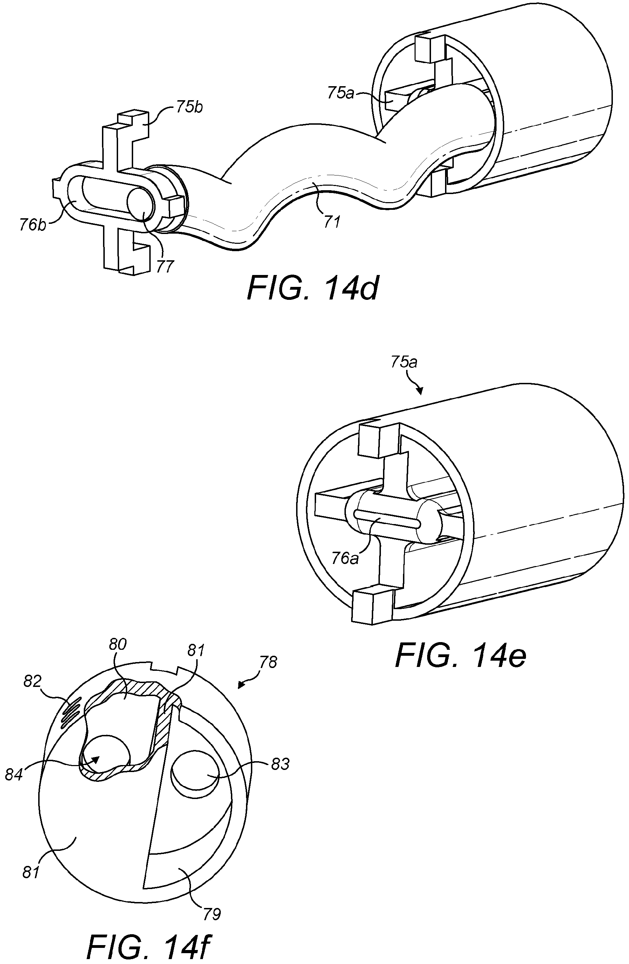

As shown in detail in Figures 14d and 14e, the flow control component 70 further comprises support elements 75a, b which are fixed in position relative to the stator housing 72 and to the flow channel housing 78. As shown, the rotor 71 is rotatably mounted by means of an elongated slot 76b formed in the support element 75b, and by means of a bearing element strip 76a formed in the mouthpiece support element 75a. As shown in Figure 14d, the end of the rotor 71 comprises a projecting element 77 which is movably located within the elongated slot 76b. In use, as the rotor 71 rotates, the projecting element 77 at the end of the rotor oscillates back and forth to obscure part of the elongated slot 76b. The projecting element may comprise bearings to facilitate rotation of the rotor 71. The other end of the rotor moves back and forth along the bearing element strip 76a as the rotor rotates.

The mouthpiece support element 75a is shown in detail in Figure 14e. A pathway for gaseous flow is defined from the lumen of the stator housing 72 around the bearing element strip 76a, and through the mouthpiece support element 75a.

The flow control component 70 further comprises a flow channel housing 78, which is shown in isolation in Figure 14f. The flow channel housing 78 is formed to define separate channels 79, 80 for smoke and air. As shown, the smoke flow channel 79 and air flow channel 80 are separated by an L-shaped barrier 81. By means of the L-shaped barrier 81, smoke from the combustion of tobacco may be drawn into the smoke flow channel 79 but not the air flow channel 80.

Air inlets 82 are formed at the periphery of the cigarette 1 to permit air to enter the air channel 80. The air inlets comprise a plurality of holes through one side of the flow channel housing 78. The air inlets further comprise holes formed in the tipping paper 6 in alignment with the holes in the flow channel part 78. In some implementations, the tipping paper may be formed from air permeable material so that air permeates through the paper.

The flow channel housing 78 further comprises first and second openings 83, 84 which are aligned with the elongated slot 76a of the support element 75a. The first opening 83 is located at the end of the smoke flow channel 79, and the second opening 84 is located at the end of the air flow channel 80.

In use, when the smoker draws on the smoking article 1, the cavities 74 formed between the rotor 71 and the internal surface 73 of the stator housing 72 are drawn towards the mouthpiece support element 75a of the flow control component 70, causing the rotor 71 to rotate. As the rotor 71 rotates, the end of the rotor moves back and forth along the elongated slot 76a thereby alternately aligning with, and thus obstructing, the first and second openings 83, 84 in the flow channel housing 78.

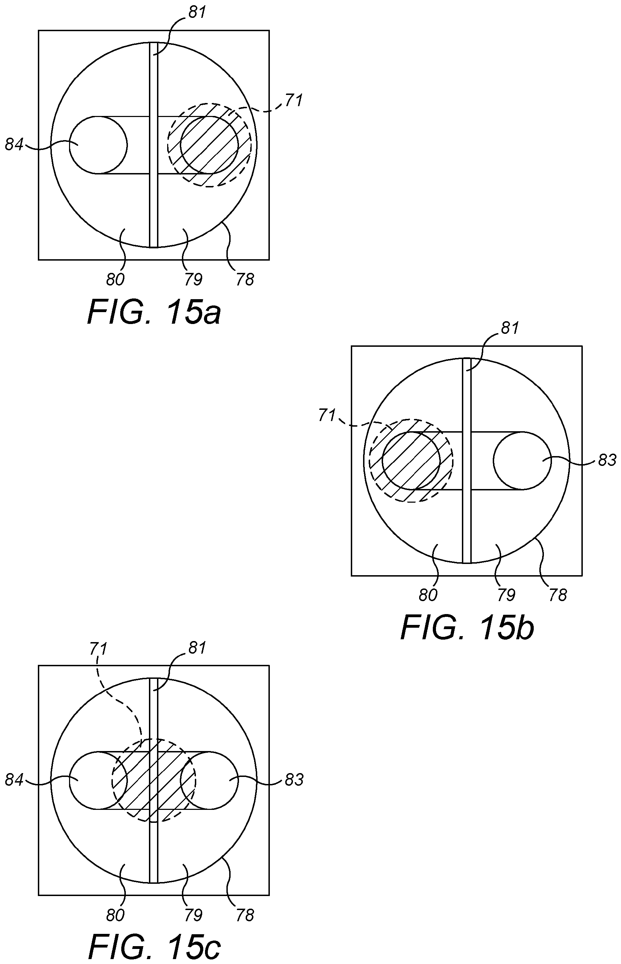

Figures 15a, 15b, and 15c illustrate diagrammatically the position of the rotor 71 relative to the flow channel housing 78. When the rotor 71 is aligned with the first opening 83 as shown in Figure 15a, a flow pathway for air is defined from the air inlets 82, through the air flow channel 80, through the second opening 84, and into the stator housing 72 of the progressive cavity assembly. Air can thus be drawn through the air flow path by the user so as to contact and drive rotation of the rotor 71. Air drawn along the stator housing 72 passes around the bearing element strip 76a, through the mouthpiece support element 75a, and into the user's mouth.

When the rotor 71 is in the position of Figure 15a, the first opening 83 is obstructed by the rotor 71 and thus smoke cannot pass into the stator housing 72. Also, smoke is prevented from passing into the air channel 80 by the barrier 81. As a result, in this position, the flow drawn from the cigarette 1 is substantially or entirely air.

When the rotor 71 is aligned with the second opening 84 in the flow channel housing 78 as shown in Figure 15b, a flow pathway for smoke is defined from the smoke flow channel 79 and through the first opening 83 and into the stator housing 72 of the progressive cavity assembly. Smoke can thus be drawn through the smoke flow path by the user so as to contact and drive rotation of the rotor 71. Smoke drawn along the stator housing 72 passes around the bearing element strip 76a, through the mouthpiece support element 75a, and into the user's mouth.

When the rotor 71 is in the position of Figure 15b, the second opening 84 is obstructed by the rotor 71 and thus air cannot pass into the stator housing 72. Also, air is prevented from passing into the smoke channel 80 by the barrier 81. As a result, in this position, the flow drawn from the cigarette 1 is substantially or entirely smoke.

In use, the user draws on the cigarette and the resulting flow drives rotation of the rotor 71. Flow in the form of smoke or air reaches the rotor 71 and drives rotation whether the rotor 71 is aligned with the first opening 83 as in Figure 15a or with the second opening 84 as in Figure 15b. In the intermediate position, as shown in Figure 15c, there is a flow path for smoke through the first opening 83, and also a flow path for air through the second opening 84. When the rotor is in this position both air and smoke reach and turn the rotor 71 before passing into the user's mouth.

Thus, as the user draws on the cigarette, the rotor 71 alternately aligns with the air and smoke channels 79, 80. In this way, the flow control component acts as a valve which repeatedly switches flow between the smoke and air pathways. Thus, the smoker is provided with a pulsed flow comprising alternating and overlapping smoke and air pulses.

Many variations of the flow control component 70 are possible. For example, by altering the size and/or shape of the openings 83, 84, the characteristics of the air and smoke pulses may be varied.

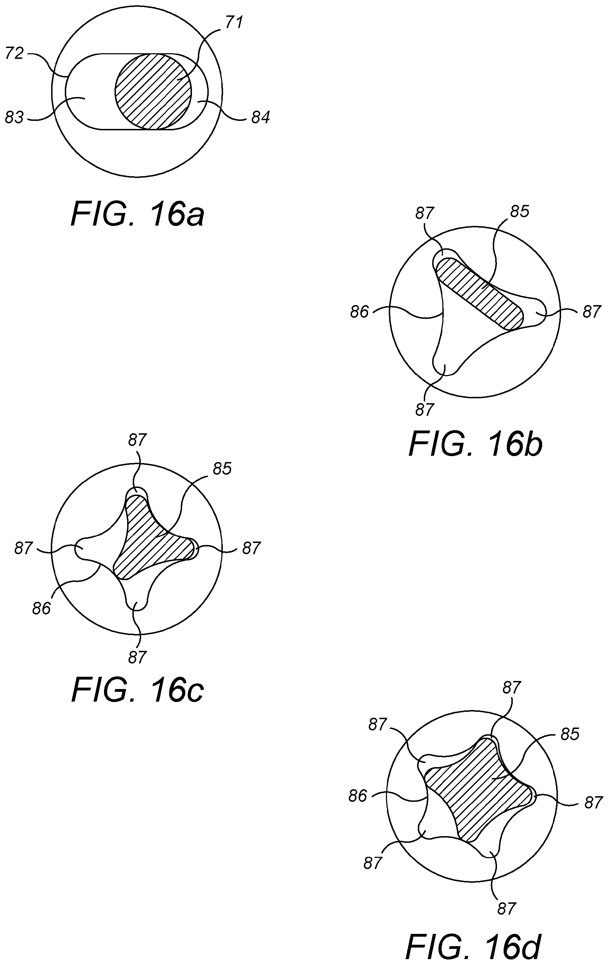

In the embodiment shown in Figures 14 and 15, the rotor 71 is circular in cross-section, and the internal surface 73 of the stator housing 72 comprises a double lobed helix. In general, any progressive cavity arrangement may be used in which the internal surface 73 of the housing 72 comprises one lobe more than the rotor 71. For example, the flow control component may comprise a double lobed rotor located within a triple lobed cavity, or a triple lobed rotor located within a quadruple lobed cavity, or a quadruple lobed rotor located within a quintuple lobed cavity. These alternative arrangements may be utilised to provide additional flow paths. For example, in addition to air and smoke flow paths, a third flow path comprising a flavourant may be provided by the use of a double lobed rotor located within a triple lobed cavity. In the case of double, triple, or quadruple lobed rotors, the flow channel housing, and in particular the number and position of openings 83, 84 must be modified such that rotation of the rotor causes the sequential obstruction and unblocking of the openings. Figure 16a shows the configuration of the rotor 71, stator housing 72 and openings 83, 84 in the case of the single lobed rotor shown in Figures 14 and 15. Figures 16b, 16c, and 16d show the configuration of the rotor 85, stator housing 86 and openings 87 in the case of double, triple, and quadruple lobed rotors, respectively.

Embodiments comprising progressive cavity assemblies are positive displacement devices. This means that substantially no flow is drawn through the device in the absence of rotation of the device. As a result, the lag time between the smoker commencing the draw and perceiving the resulting pulsed flow is minimised.

Furthermore, embodiments comprising progressive cavity assemblies have been found to generate vibration as they rotate. Vibration of the devices may be enhanced if required. For example, the rotor 71 may be eccentrically weighted, and imbalanced forces may be further tuned by alteration of the rotor diameter and/or pitch.

Many other variations of the flow control components 5, 26, 50, 70 are possible. For example, although the rotatable elements 7, 29, 51, 71 are described above as driven by the air and/or smoke flow drawn by the smoker, alternatively they may be driven by other means. For example, a small electric motor and battery may be provided to rotate the rotatable elements 7, 29, 51, 71. Suitable motors, batteries and configurations for actively driving the rotatable elements will be apparent to those skilled in the art.

Further, although movable members in the form of rotatable elements are described above, alternative flow control components to selectively control the passage of one or more flows are also contemplated. For example, in some embodiments, the flow control component may comprise one or more solenoid valves, controlled by an electrical current through a solenoid. The valve(s) may be driven to repeatedly switch a single flow on and off. Thus, in some implementations, a flow delivery article may provide only a single flow, e.g: one of smoke, air, or a mixture thereof, and may includes a valve to repeatedly switch this flow on and off, so as to provide an intermittent pulsed stream. In other flow delivery articles, a valve may be provided to repeatedly switch between different flows, e.g: to repeatedly switch between smoke and air.

Thus, according to various embodiments, a smoking article is provided in which smoke is provided in a pulsed stream. The pulsed smoke flow provides a different smoking experience, which may be desirable to consumers.

During constant stimulation from sources, such as temperature, flavour or aroma, the body tends to become desensitised. For example, the first bite of an apple, or the first sip of wine can have stronger flavour than the fourth. Changes in sensation such as temperature may also be sensed more acutely when the change occurs quickly, such as stepping into a sauna, or off an aeroplane into a very different climate.

It is thought that surfaces impacted by smoke pulses from the smoking article 1 (e.g: the oral cavity and upper respiratory tract, primarily at the back of the mouth/throat) may be repeatedly refreshed by intervening air pulses. In this way, desensitisation of the impact surfaces to smoke may be reduced, and the sensory experience of smoking is modified.

Many further variations of the flow control components described above are possible. For example, although flow control components are generally shown in the figures positioned between a tobacco rod 2 and a filter rod 3, alternatively, a flow control component may be formed within a filter rod, for example positioned between two filter rod components. In some embodiments, the flow control component may be positioned at the extreme mouth end of the smoking article, for example, adjacent to the filter rod component.

The flow control components may for example for example be formed from a suitable biodegradable material such as polyethylene (PE), polyhydroxyalkanoates (PHA), polylactides (PLA), polyvinyl alcohols (PVOH), or starch-based materials. Other materials such as polyamides, polyether ether ketones (PEEK), polyurethanes (PU), polyoxymethylene (POM), cellulose-based materials, or other suitable materials may also be used. In some embodiments, the components may comprise carbon.

According to various embodiments of the invention, exemplary flow control components may generate a pulsed flow with a pulse frequency of between 3 Hz and 1000 Hz. In embodiments, a pulse frequency in the range 50 Hz to 150 Hz, such as between 60 Hz and 100 Hz, or approximately 70 Hz, or approximately 80 Hz may be generated. In embodiments, a pulse frequency in the range 30 Hz to 1000 Hz, such as between 50 Hz and 200 Hz, or such as between 60 Hz and 70Hz, may be generated. In some examples, the vibration may result in a displacement of ± 0.1 mm.

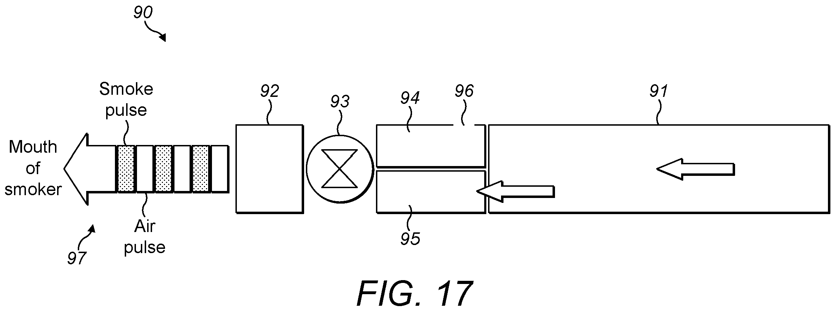

Figure 17 schematically illustrates another example of a flow delivery article in the form of smoking article 90. As shown, this smoking article 90 comprises a tobacco rod component 91 and a single filter plug component 92. Smoking article 90 includes a flow control component comprising a flow switch 93. Flow switch 93 comprises one or more valves, e.g: solenoid valve(s), which are driven to repeatedly switch between air and smoke flow. Suitable means for actively driving the flow switch to repeatedly switch between smoke and air e.g. by way of an electrical signal, will be apparent to those skilled in the art. As illustrated, the flow switch 93 is arranged to alternately switch between flows provided through an air channel 94 and a smoke channel 95. Similar to the air and smoke channels of the smoking article 1 of Figure 1, the air channel 94 receives air from an air inlet 96 at the periphery of the smoking article 90 and the smoke channel 95 receives smoke from the tobacco rod 91. A barrier may be provided to separate the air and smoke channels 94, 95.

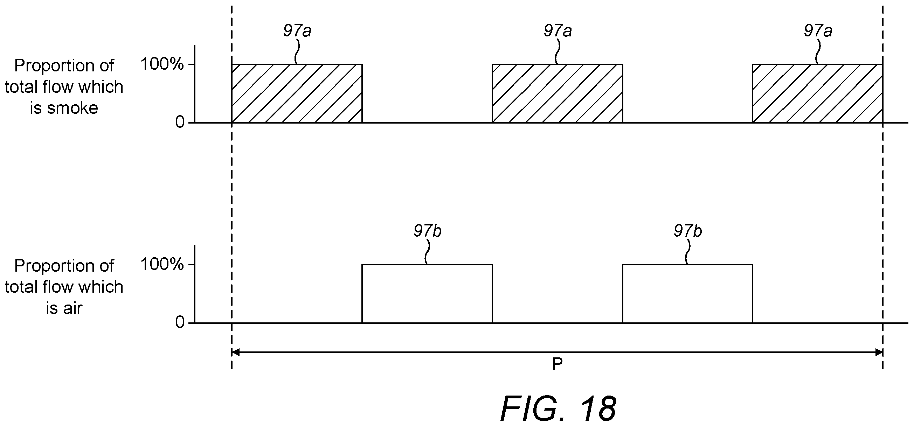

During a draw on the smoking article 90, the flow switch 93 repeatedly switches between air and smoke flow so that a pulsed flow is provided at the output of the flow switch 93, which passes through the filter component 92 and into the mouth of the smoker. As shown in Figure 18, the pulsed flow 97 comprises alternating smoke and air pulses.

Figure 18 separately illustrates the smoke and air flow components of the pulsed flow 97 during a period P. As shown, the smoke flow comprises a plurality of smoke flow peaks 97a, and the air flow comprises a plurality of air flow peaks 97b.

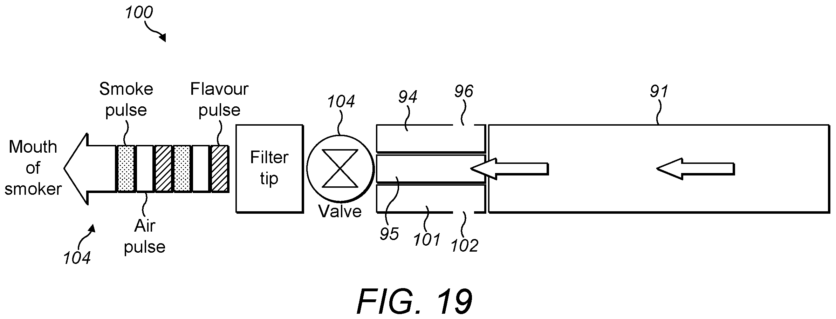

Figure 19 shows another smoking article 100. Smoking article 100 is the same as the smoking article 90 of Figure 17, apart from the differences explained below. The same reference numerals are retained for corresponding features.

Referring to Figure 19, the flow switch 104 of smoking article 100 switches between three flow inputs rather than two. The smoking article 100 has first and second air channels 94, 101 having air inlets 96, 102 at the periphery of the smoking article 100. The second air channel 101 includes an air-flavouring element (e.g: one or more flavour beads) to impart flavour (e.g: menthol flavour) to air drawn through the channel 102. As shown, the smoking article 100 also includes a smoke channel 95, which receives smoke from the tobacco rod 91.

The flow switch 104 is controlled so that a pulsed flow 103 comprising a recurring triplet of pulses is provided during a draw on the smoking article 100, as shown in Figure 19. As shown, each triplet comprises a smoke pulse drawn from the tobacco rod 91, an air pulse drawn from the air inlet 96, and a flavour pulse drawn from the inlet 102 and flavoured in the channel 101.

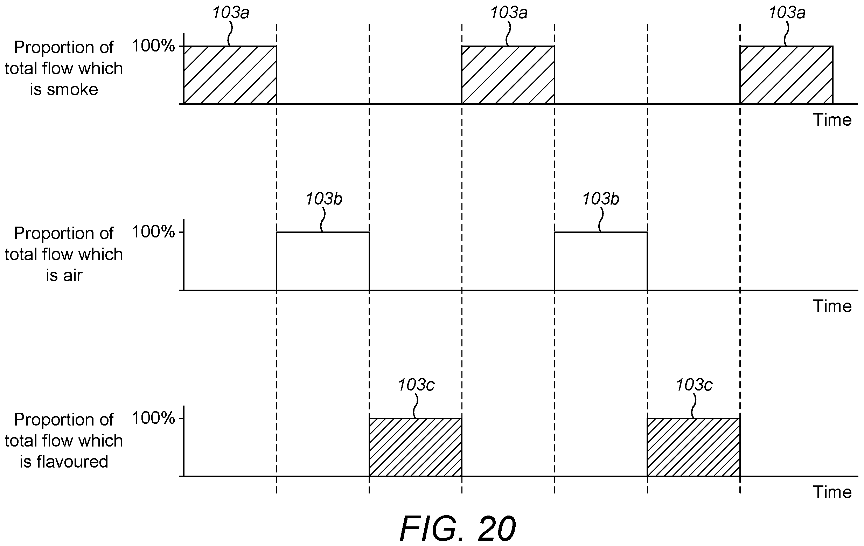

Figure 20 separately illustrates the smoke, air and flavour components of the pulsed flow 103. As shown, the smoke flow comprises a plurality of smoke flow peaks 103a, the air flow comprises a plurality of air flow peaks 103b and the flavour flow comprises a plurality of flavour flow peaks 103c.

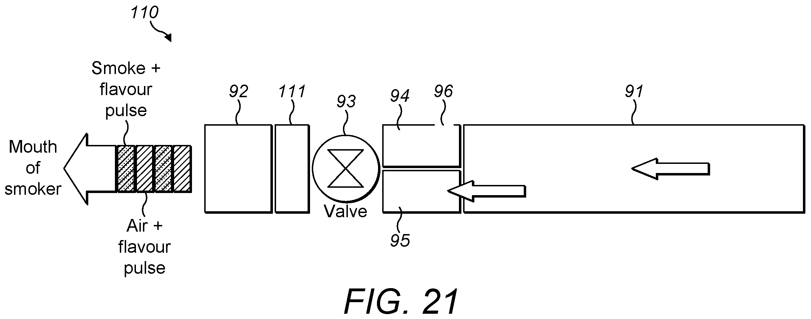

Figure 21 shows a further smoking article 110. The smoking article 110 is substantially the same as smoking article 90 of Figure 17, except that smoking article 110 includes a flavour delivery portion 111 arranged between flow switch 93 and filter component 92, which adds flavour to pulses provided at the output of flow switch 93. The flavour delivery portion may comprise a flavourant such as menthol, contained within an enclosure having a flow inlet and a flow outlet. In this way, flow drawn from the output of flow switch 93 enters the delivery portion 111, becomes flavoured, and then passes through the filter component 111 and into the mouth of the smoker. Thus, the pulsed flow provided at the output of the smoking article 110 comprises alternating pulses of flavoured smoke and flavoured air.

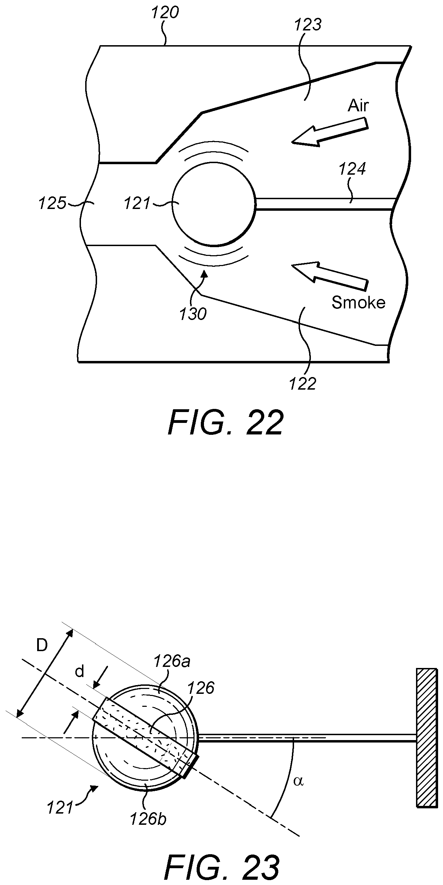

Figure 22 illustrates another example of a flow control component 120. As shown, flow control component 120 comprises a movable member 121, positioned within the path of smoke and/or air drawn through the flow control component 120. The movable member 121 is adapted so that the smoke/air flow drives movement of the movable member 121. The resulting motion preferentially directs fluid flow alternately from either the smoke or air channels 122, 123.

As shown, the movable member of Figure 22 comprises a cylindrical element which is movably cantilevered on support 124. The support is formed of a flexible, resilient material so as to allow movement of the movable member relative to a flow outlet 125. In embodiments, a part such as the part 9 of Figure 4a may be provided upstream of the movable member 121, to define separate air and smoke channels, and an end of the support 124 may be anchored to a central part of the end region 22a of the part 9.

In embodiments, the movable member may comprise a spherical ball rather than a cylindrical element. The support 124 may have a width adapted to provide a barrier to separate the smoke and air channels 122, 123.

The member 121 and support 124 may be is formed of a suitably light material so that they can be readily moved by the smoke/air flow. Thus, in use, smoke and air drawn from through the smoke and air channels 122, 123 jostles the movable member 121 about. In embodiments, the support may comprise a spring.

The member 121 is movable in a direction towards the top of the page of Figure 22, into a position in which air flow from the air channel 123 is restricted so that flow is preferentially directed from the smoke channel through the outlet 125. The element 124 is also movable in a direction towards the bottom of the page of Figure 22, into a position in which smoke flow from the smoke channel 122 is restricted, so that flow is preferentially directed from the air channel through the outlet 125.

In use, smoke and air flow drives movement of the movable member 121 between these two positions, so that a pulsed flow is provided through the outlet 125. As shown in Figure 22, the movable member 121 moves in a constricted region 130 which provides a venturi effect which increases the velocity of flow in this region.

In embodiments, the movable member 121 may comprise an aerodynamic element adapted to vortex shed, flutter, or otherwise create a dynamic flow instability. In this way, the smoke/air flow may drive continual motion of the movable member which preferentially directs flow from the smoke/air channels. Parameters of the support 124 and aerodynamic element 121 (e.g: the masses of the aerodynamic element 121 and/or the elasticity of the support 124) may be selected to obtain a resonant system, e.g: a simple harmonic resonant system.

In some implementations, the ball or cylinder suspended within the flow sheds vortexes on opposite sides of its surface at a certain velocity of smoke flow. These vortexes may shed at predictable frequency, proportional to the speed of the flow. As a consequence of the shedding of each vortex, there is a corresponding pressure change, resulting in a lift force effect acting perpendicular to the flow of the fluid. Because the vortex shedding occurs on opposing sides of the ball/tube in alternate sequence, the corresponding force is therefore approximately sinusoidal, imparting a regular perturbation force. The frequency of the perturbation force can be matched with the resonant frequency of the spherical member and support, creating an enhanced resonance.

It is thought that the phenomenon of vortex shedding may also be responsible for the simulated "swimming" motion of known fishing lures as they are drawn through water, as described for example in US2002/0194770, US2005/0193620 and US2009/0126255. In some embodiments, the member 121 and support 124 of the flow control component 120 may be formed from a similar material to such fishing lures, e.g: a suitable soft plastic material, and may be similarly shaped, so as to "swim" in the flow of smoke and air. In some embodiments, the member 121 and support 124 may be integral with one another. In some embodiments, the support is formed from a soft, floppy material to loosely tether the movable member 121.

Figure 23 shows a variation in which the movable member 121 comprises a ball 125 having a surface adapted to provide enhanced movement in the flow of smoke and air. As shown, the ball 125 has a raised ridge 126 which runs circumferentially around its diameter. The ridge 126 divides the ball into two halves 126a, 126b. One half 126a has a smooth (e.g: polished) surface and the other half 126b has a rougher, more uneven, surface. As a result, opposing sides of the ball have different aerodynamic properties. As shown, the ball 125 is tethered so that the ridge 126 makes an angle α (e.g: 45°) with the support 124. The differing surface textures on opposing sides may give rise to enhanced movement. In some implementations, enhanced movement may results from the different surface textures causing uneven and turbulent air flow, causing vortices to shed away at varying positions around the surface area of the ball. As the ball is jostled about it gives rise to further instability, and the process is continued and reinforced. In some embodiments, the nature and periodicity of the vortex shedding may vary depending on temperature, humidity and velocity of the smoke flow.

In some embodiments, the movable member may repeatedly impact the walls of the region 110 and in this way generate vibration of the flow control component. This vibration may be imparted to the filter and/or tobacco rod of the smoking article so as to be perceived tactually by the smoker's lips or fingers.

## Claims

1. A flow delivery article (1) to deliver a gaseous flow to the mouth of a user, **characterised in that** the flow delivery article comprises:
a flow control component (5) to provide at least one flow during a draw on the flow delivery article, said at least one flow comprising a plurality of flow pulses each having a respective flow peak; and
a plurality of flow pathways (11, 12), wherein the flow control component is arranged to repeatedly change the relative amount of flow through said pathways.

2. A flow delivery article (1) according to claim 1, wherein the flow control component (5) is configured to control the passage of flow to provide at least three consecutive flow pulses during the draw on the flow delivery article.

3. A flow delivery article as claimed in claim 1, wherein said flow control component is configured to provide a plurality of different flows (103), each flow comprising a respective plurality of flow peaks (103a, 103b, 103c).

4. A flow delivery article as claimed in claim 2, wherein each of said three consecutive flow pulses is different.

5. A flow delivery article as claimed in any preceding claim, wherein the flow control component comprises a movable member (7), and wherein the movable member is arranged so that received flow causes the movable member to move.

6. A flow delivery article as claimed in claim 5 wherein the movable member comprises a rotary member (17) configured so that rotation of the rotatable member changes the relative amount of flow through said flow pathways.

7. A flow delivery article as claimed in claim 6, wherein the rotary member comprises a flow-driven rotary member comprising one or more flow-receiving portions (16) configured to cause rotation of the rotary member in response to receiving flow.

8. A flow delivery article as claimed in any of claims 5 to 7, wherein the flow control component (70) comprises a progressive cavity device.

9. A flow delivery article as claimed in any of claims 6 to 7, wherein the movable member comprises a first flow-receiving portion (39) to cause rotation of the rotary member (29) in response to receiving a first flow, and a second flow-receiving portion (40) to cause rotation of the rotary member in response to receiving a second flow, wherein the first flow pathway is a flow pathway (27) for the first flow and the second flow pathway is a flow pathway (28) for the second flow.

10. A flow delivery article as claimed in claim 9, wherein the first flow-receiving portion comprises a helical element (57) and the second flow-receiving portion comprises a helical element (58).

11. A flow delivery article as claimed in claim 9, wherein the first flow-receiving portion comprises one or more paddles (41) and the second flow-receiving portion comprises one or more paddles (42).

12. A flow delivery article as claimed in claim 5, wherein the movable member comprises a fan (10) arranged so that received flow causes the fan to rotate; and optionally, further comprising a support member (14) to support the movable member in the flow.

13. A flow delivery article as claimed in claim 5 or 12, wherein the movable member comprises a rotatable opening (13) configured to permit flow through the first flow pathway in a first rotational position and through the second flow pathway in a second rotational position.

14. A flow delivery article as claimed in any preceding claim, wherein the flow control component is configured to provide a flow comprising a train of pulses and further comprising a flavouring component to flavour at least some of said pulses.

15. A flow delivery article as claimed in any preceding claim, comprising a smoking article.

16. A flow delivery article as claimed in claim 15, wherein the smoking article comprises a tobacco rod (2) and a filter (3, 4), wherein the filter comprises the flow control component (5); and optionally, wherein the flow control component is positioned within the filter.

## Patentansprüche

1. Flussfreisetzungsartikel (1) zur Abgabe einer gasförmigen Strömung an den Mund eines Benutzers, **dadurch gekennzeichnet, dass** der Flussfreisetzungsartikel folgendes umfasst:
eine Flusssteuerungskomponente (5) zur Bereitstellung mindestens einer Strömung während eines Zugs an dem Flussfreisetzungsartikel, wobei die mindestens eine Strömung eine Vielzahl von Strömungsimpulsen umfasst, die jeweils eine jeweilige Strömungsspitze aufweisen; und
eine Vielzahl von Strömungswegen (11, 12), wobei die Flussteuerungskomponente dazu angeordnet ist, die relative Strömungsmenge durch die Wege wiederholt zu ändern.

2. Flussfreisetzungsartikel (1) nach Anspruch 1, wobei die Flusssteuerungskomponente (5) dazu ausgelegt ist, den Strömungsdurchfluss zu steuern, um mindestens drei aufeinanderfolgende Strömungsimpulse während des Zugs an dem Flussfreisetzungsartikel bereitzustellen.

3. Flussfreisetzungsartikel nach Anspruch 1, wobei die Flusssteuerungskomponente zur Bereitstellung einer Vielzahl von unterschiedlichen Strömungen (103) ausgelegt ist, wobei jede Strömung eine jeweilige Vielzahl von Strömungsspitzen (103a, 103b, 103c) umfasst.

4. Flussfreisetzungsartikel nach Anspruch 2, wobei jeder der drei aufeinanderfolgenden Strömungsimpulse unterschiedlich ist.

5. Flussfreisetzungsartikel nach einem der vorhergehenden Ansprüche, wobei die Flusssteuerungskomponente ein bewegliches Element (7) umfasst und wobei das bewegliche Element so angeordnet ist, dass eine empfangene Strömung eine Bewegung des beweglichen Elements verursacht.

6. Flussfreisetzungsartikel nach Anspruch 5, wobei das bewegliche Element ein Drehelement (17) umfasst, das so ausgelegt ist, dass eine Drehung des Drehelements die relative Strömungsmenge durch die Strömungswege verändert.

7. Flussfreisetzungsartikel nach Anspruch 6, wobei das Drehelement ein durch Strömung angetriebenes Drehelement umfasst, das ein oder mehrere Strömungsaufnahmeabschnitte (16) umfasst, die dazu ausgelegt sind, als Reaktion auf eine erhaltene Strömung eine Drehung des Drehelements zu verursachen.

8. Flussfreisetzungsartikel nach einem der Ansprüche 5 bis 7, wobei die Flusssteuerungskomponente (70) eine Exzenterschneckenvorrichtung umfasst.

9. Flussfreisetzungsartikel nach einem der Ansprüche 6 bis 7, wobei das bewegliche Element einen ersten Strömungsaufnahmeabschnitt (39), um eine Drehung des Drehelements (29) als Reaktion auf eine erhaltene erste Strömung zu verursachen, und einen zweiten Strömungsaufnahmeabschnitt (40), um eine Drehung des Drehelements als Reaktion auf eine erhaltene zweite Strömung zu verursachen, umfasst, wobei der erste Strömungsweg ein Strömungsweg (27) für die erste Strömung ist und der zweite Strömungsweg ein Strömungsweg (28) für die zweite Strömung ist.

10. Flussfreisetzungsartikel nach Anspruch 9, wobei der erste Strömungsaufnahmeabschnitt ein spiralförmiges Element (57) umfasst und der zweite Strömungsaufnahmeabschnitt ein spiralförmiges Element (58) umfasst.

11. Flussfreisetzungsartikel nach Anspruch 9, wobei der erste Strömungsaufnahmeabschnitt ein oder mehrere Paddel (41) umfasst und der zweite Strömungsaufnahmeabschnitt ein oder mehrere Paddel (42) umfasst.

12. Flussfreisetzungsartikel nach Anspruch 5, wobei das bewegliche Element ein Gebläse (10) umfasst, das so angeordnet ist, dass die erhaltene Strömung eine Drehung des Gebläses verursacht; und fakultativ ferner umfassend ein Stützelement (14) zur Unterstützung des beweglichen Elements in der Strömung.

13. Flussfreisetzungsartikel nach Anspruch 5 oder 12, wobei das bewegliche Element eine drehbare Öffnung (13) umfasst, die dazu ausgelegt ist, eine Strömung durch den ersten Strömungsweg in einer ersten Drehrichtung und durch den zweiten Strömungsweg in einer zweiten Drehrichtung zu gestatten.

14. Flussfreisetzungsartikel nach einem der vorhergehenden Ansprüche, wobei die Flusssteuerungskomponente dazu ausgelegt ist, eine Strömung bereitzustellen, die eine Impulsfolge umfasst, und ferner umfassend eine Aromakomponente zur Aromatisierung mindestens einiger der Impulse.

15. Flussfreisetzungsartikel nach einem der vorhergehenden Ansprüche, einen Rauchartikel umfassend.

16. Flussfreisetzungsartikel nach Anspruch 15, wobei der Rauchartikel einen Tabakstrang (2) und einen Filter (3, 4) umfasst, wobei der Filter die Flusssteuerungskomponente (5) umfasst; und wobei die Flusssteuerungskomponente fakultativ innerhalb des Filters angeordnet ist.

## Revendications

1. Objet de distribution (1) de flux pour distribuer un flux vers la bouche d'un utilisateur, **caractérisé en ce que** l'objet de distribution de flux comprend :
un composant de régulation (5) de flux pour fournir au moins un flux pendant une aspiration sur l'objet de distribution de flux, ledit au moins un flux comprenant une pluralité d'impulsions de flux ayant chacune un pic de flux respectif; et
une pluralité de passages (11, 12) de flux, le composant de régulation de flux étant conçu pour modifier de façon répétée la quantité relative de flux par lesdits passages.

2. Objet de distribution (1) de flux selon la revendication 1, dans lequel le composant de régulation (5) de flux est conçu pour réguler le passage de flux afin de fournir au moins trois impulsions de flux consécutives pendant l'aspiration sur l'objet de distribution de flux.

3. Objet de distribution de flux selon la revendication 1, dans lequel ledit composant de régulation de flux est conçu pour fournir une pluralité de différents flux (103), chaque flux comprenant une pluralité respective de pics (103a, 103b, 103c) de flux.

4. Objet de distribution de flux selon la revendication 2, dans lequel chacune desdites trois impulsions consécutives de flux est différente.

5. Objet de distribution de flux selon l'une quelconque des revendications précédentes, dans lequel le composant de régulation de flux comprend un élément mobile (7), et dans lequel l'élément mobile est conçu de sorte que le flux reçu amène l'élément mobile à se déplacer.

6. Objet de distribution de flux selon la revendication 5 dans lequel l'élément mobile comprend un élément rotatif (17) conçu de sorte que la rotation de l'élément rotatif change la quantité relative de flux à travers lesdits passages de flux.

7. Objet de distribution de flux selon la revendication 6, dans lequel l'élément rotatif comprend un élément rotatif entraîné par le flux comprenant une ou plusieurs parties (16) de réception de flux conçues pour provoquer la rotation de l'élément rotatif en réponse à la réception du flux.

8. Objet de distribution de flux selon l'une quelconque des revendications 5 à 7, dans lequel le composant (70) de régulation de flux comprend un dispositif à cavité progressive.

9. Objet de distribution de flux selon l'une quelconque des revendications 6 à 7, dans lequel l'élément mobile comprend une première partie (39) de réception de flux pour provoquer la rotation de l'élément rotatif (29) en réponse à la réception d'un premier flux, et une seconde partie (40) de réception de flux pour provoquer la rotation de l'élément rotatif en réponse à la réception d'un second flux, le premier passage de flux étant un passage (27) de flux pour le premier flux et le second passage de flux étant un passage (28) de flux pour le second flux.

10. Objet de distribution de flux selon la revendication 9, dans lequel la première partie de réception de flux comprend un élément hélicoïdal (57) et la seconde partie de réception de flux comprend un élément hélicoïdal (58).

11. Objet de distribution de flux selon la revendication 9, dans lequel la première partie de réception de flux comprend une ou plusieurs pales (41) et la seconde partie de réception de flux comprend une ou plusieurs pales (42).

12. Objet de distribution de flux selon la revendication 5, dans lequel l'élément mobile comprend un ventilateur (10) agencé de sorte que le flux reçu amène le ventilateur à tourner; et éventuellement, comprenant en outre un élément de support (14) pour supporter l'élément mobile dans le flux.

13. Objet de distribution de flux selon l'une quelconque des revendications 5 ou 12, dans lequel l'élément mobile comprend une ouverture rotative (13) conçue pour permettre un flux à travers le premier passage de flux dans une première position rotative et à travers le second passage de flux dans une seconde position rotative.

14. Objet de distribution de flux selon l'une quelconque des revendications précédentes, dans lequel un composant de régulation de flux est conçu pour fournir un flux comprenant un train d'impulsions et comprenant en outre un composant aromatisant pour donner un arôme à au moins une partie desdites impulsions.

15. Objet de distribution de flux selon l'une quelconque des revendications précédentes, comprenant un objet à fumer.

16. Objet de distribution de flux selon la revendication 15, dans lequel l'objet à fumer comprend un boudin de tabac (2) et un filtre (3, 4), dans lequel le filtre comprend le composant de régulation (5) de flux; et éventuellement, dans lequel le composant de régulation de flux est positionné à l'intérieur du filtre.
